# EUROPEAN PATENT APPLICATION

(11) **EP 1 402 903 A1**
(43) Date of publication of application: **31.03.2004**
(21) Application number: 02743781.3
(22) Date of filing: 02.07.2002
(51) Int. Cl.: A61K 48/00, A61K 38/00, A61K 38/02, A61K 47/48, C07K 14/03, C12N 15/00, A61P 29/00, A61P 31/00, A61P 33/00, A61P 43/00

(54) **METHOD OF TRANSPORTING PHYSIOLOGICAL POLYMER USING PROTEIN HAVING RXP REPEATED SEQUENCE**

(30) Priority: 05.07.2001 JP 2001204618
(71) Applicant: M'S Science Corporation, Chuo-ku, Kobe-shi, Hyogo 650-0047 (JP); Nagoya Industrial Science Research Institute, Nagaya-shi, Aichi 460-0008 (JP)
(72) Inventor: NISHIYAMA, Yukihiro, Aichi-gun, Aichi 470-0155 (JP); KOSHIZUKA, Tetsuo, Nagoya-shi, Aichi 466-0001 (JP)
(74) Representative: Keller, Günter, Dr.
(86) International application number: PCT/JP2002/006703
(87) International publication number: WO 2003/004065

(57) **Abstract**

The present invention provides a method for efficiently transporting useful protein, peptide, gene, etc. to a target cell such as a diseased cell, which is characterized by that a complex in which protein having a structure of peptide unit repetition represented by the formula Arg-Xaa-Pro (in the formula, Xaa is a hydrophobic or acidic amino acid residue) at the C-terminal of the protein is bound to at least one of useful protein, peptide, gene, etc. is administered to a living body.

## Description

### Technical Field

The present invention relates to a method for efficiently transporting physiological polymer such as useful protein, peptide and gene, etc. in a living body. Such a method is particularly useful in gene therapy.

### Background Art

As a result of the progress in genetic engineering in recent years, genetic abnormality that causes many diseases such as hereditary disease has been explicated at the DNA level, whereupon there have been invented therapeutic methods in which the abnormal gene is returned to normal and expressed so that the disease is treated.

In such therapeutic methods, one of the technical challenges is development of an art for transporting useful genes efficiently and safely to a target cell and expressing them.

With regard to a method for introducing genes into a cell, microinjection, precipitation with calcium phosphate, cation liposome, virus vector, electroporation, etc. have been usually applied. However, there are disadvantages that those methods are troublesome and also have cytotoxicity. In addition, it takes as long as 12 to 80 hours to confirm the expression of the desired genes after introduction of genes.

In order to overcome such disadvantages, there has been developed a method for introducing useful protein directly into a cell by using protein which has intercellular transportation ability. With regard to the protein having an intercellular transportation ability, there have been known HIV-1 TAT (Vives, E., Brodin, P. and Lebleu, B. (1997), A truncated HIV-1 Tat protein basic domain rapidly translocates through the plasma membrane and accumulates in the cell nucleus, *J. Biol. Chem.* 272, 16010-7), antennapedia (Derossi, D., Joliot, A/H/. Chassaing, G. and Prochiantz, A. (1994), The third helix of the Antennapedia homeodomain translocates through biological membrane, *J. Biol. Chem.* 269, 10444-50), etc. When those proteins are used, however, there is a problem that inactivation or denaturation of the useful protein to be introduced into a cell is resulted.

### Disclosure of the Invention

The present invention provides a method for binding intercellular transportation protein to physiological polymer such as useful protein, peptide or gene and introducing the resulting complex into a target cell such as a diseased cell, wherein the above-mentioned problems are solved.

The present inventors have found that useful protein, peptide or gene is able to be intercellularly transported without depending upon energy such as ATP, when protein having repetition of tripeptide represented by the formula Arg-Xaa-Pro (in the formula, Xaa is a hydrophobic or acidic amino acid residue) with abundant positively charged residues at the C-terminal, such as HSV US11 protein which is a US11 gene product of HSV, interacts with negative charge of cytoplasmic membrane.

The present inventors conducted intensive investigations, and have created a method in which protein having a structure of peptide unit repetition represented by the formula Arg-Xaa-Pro (in the formula, Xaa is a hydrophobic or acidic amino acid residue) at the C-terminal of the protein is bound to at least one of physiological polymer such as (a) useful protein, (b) peptide and (c) gene, etc., whereby at least one of the physiological polymer such as (a) useful protein, (b) peptide and (c) gene, etc. is/are introduced into a target cell such as a diseased cell.

Thus, the present invention relates to
(1) A method for transporting at least one of (a) useful protein, (b) peptide and (c) gene in a living body, which is characterized by that at least one of (a) useful protein, (b) peptide and (c) gene is/are bound to protein having a structure of peptide unit repetition represented by the formula Arg-Xaa-Pro (in the formula, Xaa is a hydrophobic or acidic amino acid residue) at the C-terminal of the protein and the resulting complex is administered to a living body.
(2) The method mentioned in the above (1), wherein the gene is inserted into a plasmid.
(3) The method mentioned in the above (1), wherein transport in a living body is intercellular transport.
(4) The method mentioned in the above (3), wherein intercellular transport is transport to a target cell.
(5) The method mentioned in the above (4), wherein the target cell is a diseased cell.
(6) The method mentioned in the above (1), wherein the protein is US11 protein of herpes simplex virus.
(7) The method mentioned in the above (1), wherein the useful protein is a cytokine or interferon-α, -β, -γ or -ω.
(8) Use of protein having a structure of peptide unit repetition represented by the formula Arg-Xaa-Pro (in the formula, Xaa is a hydrophobic or acidic amino acid residue) at the C-terminal of the protein for transporting at least one of (a) useful protein, (b) peptide and (c) gene in a living body.
(9) A transport agent for transporting at least one of (a) useful protein, (b) peptide and (c) gene in a living body, which comprises a complex in which protein having a structure of peptide unit repetition represented by the formula Arg-Xaa-Pro (in the formula, Xaa is a hydrophobic or acidic amino acid residue) at the C-terminal of the protein is bound to at least one of (a) useful protein, (b) peptide and (c) gene.
(10) A complex in which protein having a structure of peptide unit repetition represented by the formula Arg-Xaa-Pro (in the formula, Xaa is a hydrophobic or acidic amino acid residue) at the C-terminal of the protein is bound to at least one of (a) useful protein, (b) peptide and (c) gene.
(11) The complex mentioned in the above (10), wherein the gene is inserted into a plasmid.
(12) The complex mentioned in the above (10), wherein the protein is US11 protein of herpes simplex virus.
(13) A method for the treatment of human beings or animals, which is characterized by that a complex in which protein having a structure of peptide unit repetition represented by the formula Arg-Xaa-Pro (in the formula, Xaa is a hydrophobic or acidic amino acid residue) at the C-terminal of the protein is bound to at least one of (a) useful protein, (b) peptide and (c) gene is administered to a patient.

### Best Mode for Carrying Out the Invention

In the present invention, protein having repetition of tripeptide represented by the formula Arg-Xaa-Pro (in the formula, Xaa is a hydrophobic or acidic amino acid residue; hereinafter, it is abbreviated as RXP) with abundant positively charged residues at the C-terminal of the protein is used. There is no particular limitation for Xaa so far as it is a hydrophobic or acidic amino acid residue, and examples of the hydrophobic amino acid residue are alanine, isoleucine, leucine, methionine, phenylalanine, proline, etc., while examples of the acidic amino acid residue are aspartic acid glutamic acid.

With regard to the protein used in the present invention, protein having RXP repetition at the C-terminal may be used, and also an arbitrary protein where RXP repetition is introduced into the C-terminal may be used. Introduction of RXP repetition into the C-terminal may be carried out by a known method per se. The numbers of RXP repetition at the C-terminal may be about 10 to 40.

A preferred example of the protein having RXP repetition at the C-terminal is HSV US11 protein which is a US11 gene product of herpes simplex virus (hereinafter, abbreviated as HSV). The number of RXP repetition at the C-terminal of HSV US11 is 19.

The method for the production of pure HSV US11 protein is not limited to the following method and may be carried out according to known methods. A preferred method for the production of pure HSV US11 protein is shown below.

An open reading frame (ORF) of HSV US11 is located between the positions 147247 and 147699 of a nucleotide of HSV-2 genome. A base sequence coding for HSV US11 protein is amplified from HSV-2186 genomic DNA by polymerase chain reaction (hereinafter, abbreviated as PCR method), wherein US11-F (ATTAGGATCCATGGCATCCGGGGTTTCC) and US11-R (TTATGCGGCCGCCTAGGCAAGCCCGCGGGT) are used as a forward primer and a reverse primer, respectively.

The resulting PCR product is cleaved by Sal I and Not I and inserted into a frame of *Escherichia coli* expression vector pET-28a (manufactured by Novagen) to prepare a plasmid pET-28a-US11 followed by being cloned. Lac operator and IPTG-inducible tac promoter are used to control the expression. The plasmid is transformed into *Escherichia coli* BL21DE3 strain (manufactured by Novagen) to express a large quantity of 6xHis-tagged US11 fusion protein. The 6xHis-tagged US11 fusion protein expression cells are dissolved and separated in PBS using ultrasonic wave, the cell pieces are removed by centrifugation, and the 6xHis-tagged US11 fusion protein is eluted with a fraction containing 300 mM imidazole, thus pure HSV US11 protein is obtained.

There is no particular limitation for useful protein which may be used in the present invention, and protein which is useful for the prevention or treatment of diseases of human beings or animals is preferred. A pharmaceutically effective, useful protein which has been difficult to be incorporated into cells such as diseased cells is particularly preferred. Any protein may be used as useful protein, and its examples are a cytokine, interferon-α, β, γ or ω, interferon of vertebrates, and animal-derived interferon such as canine interferon (α, β and γ type) and feline interferon ω. Other examples are hemagglutinin (hemagglutinative agent) of influenza A or B virus, HA 2 component of hemagglutinin of influenza A or B virus and peptide analogs thereof, M2 protein of influenza A virus and HEF protein of influenza C virus.

With regard to the useful protein, pure one can be procured according to a known method. Useful protein can be prepared by such a manner that, taking its properties such as stability to salt, hydrophobicity and biological specificity into consideration, impurities such as protease are removed by various types of chromatography, and the aimed protein is recovered and concentrated. Examples of the chromatography are gel filtration chromatography, ion-exchange chromatography, reversed phase chromatography, affinity chromatography and hydrophobic chromatography.

With regard to the peptide which may be used in the present invention, any peptide may be used so far as it is a peptide useful in a living body, and such peptide is obtained, for example, by modifying the above-mentioned useful protein into peptide form, and peptide still maintaining the utility of the useful protein is preferred. There is preferably listed a peptide where protein useful for the prevention or treatment of diseases of human beings or animals is modified. With regard to a means for producing peptide by modification of protein, a method for decomposition of protein such as hydrolysis may be exemplified. It may also be a peptide prepared by mixing individual amino acids. Although a peptide is usually produced by introducing amino acids step by step according to an organic chemical synthetic method, it may also be produced by other methods such as enzymatic hydrolysis of natural protein or peptide synthesis by an enzymatic method.

With regard to a method of introducing amino acids step by step according to an organic chemical synthetic method, solid phase peptide synthesis or liquid phase peptide synthesis has been known, and they are mentioned in detail, for example, in *Fundamentals and Experiments of Peptide Syntheses* by Nobuo Izumiya, et al.(Maruzen).

With regard to a method for producing peptide by enzymatic hydrolysis of natural protein, for example, a method where milk casein is hydrolyzed with trypsin or a method where corn protein is hydrolyzed with microorganism-derived thermolysin to prepare hypotensive peptide has been known (Susumu Maruyama, *Bioscience and Industry,* volume 47, pages 38 to 42, 1989).

With regard to a method for peptide synthesis by means of an enzymatic method, there are two methods - condensation and substitution - and in the former, an amine component is substituted for a carboxyl component having a free carboxyl group, and in the latter, an amine component is substituted for an esterified or amidated carboxyl component. All of them may be carried out according to a known method.

The present invention can be used for introducing gene which is useful for a living body into a living body. For example, gene which is useful for the prevention and treatment of diseases of human beings or animals or, in other words, gene which is used in gene therapy is listed. It is preferred that the gene is in a form of a plasmid. The gene to be inserted into a vector may be prepared, for example, by conducting PCR utilizing a primer DNA, which is designed and prepared on the basis of a base sequence of the original protein using, as a template, a genomic DNA of a living body (cDNA, when the living body is an eukaryote) which produces useful protein (hereinafter, referred to as original protein) expressing in a cell. cDNA can be directly amplified by means of a reverse transcriptase polymerase chain reaction (hereinafter, abbreviated as RT-PCR method) using total RNA or an mRNA fraction which is prepared from cells/tissues. Also, those produced according to a synthetic method for an oligonucleotide known *per se* may be used. To be more specific, a chemical synthesis method using a DNA synthesizer such as a DNA synthesizer model 392 (manufactured by Perkin-Elmer) utilizing a phosphoamidite method may be listed.

With regard to a method for the preparation of genomic DNA or cDNA of the living body which produces the original protein, it is appropriately selected depending upon the type of the living body and is not particularly limited. For example, it is possible to apply a method mentioned in *Molecular Cloning,* 2nd Ed. , Cold Spring Harbor Laboratory Press (1989), J. Sambrook, et al.

With regard to gene to be introduced into a target cell (such as a diseased cell), there is no particular limitation and, for example, gene for the diseases, i.e. gene which antagonistically acts on the disease, gene which supplements the deficiency in the disease, etc. may be used. With regard to the genes as such, gene which expresses the above-mentioned useful protein in a living body may be specifically listed, and its example is gene which codes for a pharmacologically active substance selected from the group consisting of cytokine, growth factor, antibody or antibody fragment, receptor to cytokine or growth factor, protein having effect of growth inhibition or cell growth suppression, enzyme, thrombus-inducing substance, agglutination-inhibiting substance, protein having fibrinolysis action, virus coat protein, bacterial antigen and parasitic antigen, tumor antigen, protein effecting on blood circulation, peptide hormone and ribonucleic acid such as ribozyme and antisense RNA.

Further, examples of representative gene to specific diseases are as follows. To inflammatory diseases, they are genes coding for SOD, anti-inflammatory cytokines and peptide antagonistically acting on cell adhesion factors; to enzyme deficiency, they are genes coding for normal enzymes; to receptor deficiency, they are genes coding for normal receptors; to viral infection, they are genes coding for thymidine kinase for killing/damaging virally infected cells, genes coding for a toxin such as diphtheria toxin, etc., and genes coding for antisense, triple helix, ribozyme, decoy, transdominant mutant, etc. which inhibit the duplication of virus; to cancer, they are genes coding for thymidine kinase for killing/damaging cancer cells, genes coding for a toxin such as diphtheria toxin, etc., genes coding for antisense, ribozyme, triple helix, etc. for inactivating the cancer genes, genes coding for cancer-suppressing genes such as p53 for normalizing the cancer cells, and genes coding for antisense, triple helix, ribozyme, etc. for inactivating the genes participating in multidrug resistance to anti-cancer agents; to familial hypercholesterolemia, they are genes coding for LDL receptors; etc.

The gene to be used in the present invention may be prepared by being inserted into a duplicable plasmid vector, a phage vector, etc. in a target cell according to a common method. In the present invention, a form in a plasmid is preferred. With regard to a vector, it is preferred to use an expression vector which is prepared in such a manner that stable mRNA is able to be generated in large quantities and the resulting mRNA is efficiently translated also in a target cell. When plural genes are introduced, it is preferred to use vectors having different origins of replication (ori) even if they are same genes or different genes.

With regard to a plasmid vector, it may have drug-resistance genes such as erythromycin-resisting gene, neomycin-resisting gene, kanamycin-resisting gene, etc. in view of being able to easily screen a transformant having a recombined vector to be prepared and also being able to stably hold the recombined vector in the target cell.

With regard to a plasmid vector used for insertion of genes, examples thereof include plasmid pET-28a, pFLAG-CMV-5a, pCDM8, pcDNAI/Amp, pcDL-SRα, BCMGSNeo, pSV2-neo, pSV-2gpt, pEF-BOS, pCEV 4, pME 18S, pKY 4, pKK 223-3, pVL 1392, pVL 1393, Ti plasmid, Ri plasmid, pBI 121, pUB 110, pUC 118, etc. The plasmid may be appropriately selected depending upon the type of the gene to be inserted, and commercially available plasmids may be used. Examples of the commercially available plasmids are pET-28a (manufactured by Novagen) and pFLAG-CMV-5a (manufactured by Novagen), etc.

With regard to a phage vector, λgt 11 phage, λgt 10 phage, etc. may be utilized. Any of them gives a recombinant vector corresponding to the parent vector used. The phage vector may be appropriately selected depending upon the gene to be inserted, and a commercially available one may also be used.

General means for the preparation of recombinant plasmids is described in *Molecular Cloning,* 2nd ed., Cold Spring Harbor Laboratory Press, (1989) J. Sambrook, et al.

With regard to an expression cassette used for the gene to be inserted, anything may be used without particular limitation so far as it is able to express the gene in a target cell. As for the expression cassette, an expression cassette which is able to express the gene in animal-derived cells may be used. Preferably, it is an expression cassette which is able to express the gene in mammalian-derived cells and, more preferably, it is an expression cassette which is able to express the gene in human-derived cells.

The expression vector usually contains a regulatory sequence to express the gene or to be advantageous for the expression. Each regulatory sequence may be native or foreign to a base sequence coding for an amino acid sequence of the gene. Such a regulatory sequence includes promoter, leader, polyadenylated sequence, propeptide sequence, enhancer, signal sequence, splicing signal, poly A-added signal, SV 40 duplicated origin and transcription terminator, although they are not limitative. Among them, it is preferred that the regulatory sequence includes at least promoter and transcription- and translation-termination signals.

With regard to a promoter, anything may be used so far as it is an appropriate promoter sequence which is a base sequence recognizable in the target cell. Examples thereof are lac promoter, SRα promoter, SV 40 promoter, LTR promoter, CMV (cytomegalovirus) promoter and HSV-TK promoter, etc.

With regard to the transcription terminator, anything may be used so far as it is a sequence which is recognizable in a target cell for completing the transcription and, for example, transcription terminator sequences of genes derived from virus, various mammals and birds may be used. To be more specific, SV 40 terminator of simian virus, etc. may be used.

The expression vector also includes a signal sequence concerning secretion of protein. As for the signal sequence, either a signal sequence of the gene to be introduced or a signal sequence of different gene may be used. For example, insulin signal sequence, α-interferon signal sequence, antibody signal sequence, etc. may be used.

The expression vector may include one or more factor(s) advantageous for expression of enzyme gene such as one or more nucleic acid sequence(s) coding for activator (such as trans-acting factor), chaperone and processing protease. Any factor which is functional in a target cell may also be used as an expression vector according to the present invention.

Protein having RXP repetition at the C-terminal forms a complex with useful protein, peptide, a plasmid into which gene is inserted, etc. by a non-covalent bond. The complex of protein having RXP repetition at the C-terminal with useful protein, peptide, a plasmid into which gene is inserted, etc. moves into nucleolus of the target cell and its surrounding cells.

In the case when a complex is formed with useful protein having a molecular weight of more than 10 kDa, protein having RXP repetition at the C-terminal is added to, for example, PBS or serum-deficient medium in an aseptic tube preferably in an amount of about 1/100 (w/w) relative to the total amount. After that, about 0.25 to 1 part by weight of the useful protein is added thereto, followed by mixing.

In the case of a plasmid into which peptide, gene or a low-molecular useful protein of not more than 10 kDa is inserted, a protein having RXP repetition at the C-terminal which is diluted to about 1/10 (w/w) with PBS is added to PBS or serum-deficient medium in an aseptic tube in an amount of about 1/100 (w/w) relative to the total amount. After that, about 100 to 500 parts by weight of peptide or low-molecular useful protein of not more than 10 kDa is added thereto, followed by mixing.

After mixing, the mixture is incubated at about 15 to 30°C or, preferably, at about 20 to 25°C for about 20 to 50 minutes or preferably about 30 to 40 minutes to form a complex. The complex prepared as such is administered to a living body. With regard to a method for the administration to a living body, a method where it is directly administered to a living body by means of injection or the like, a method where a target cell such as a diseased cell is extracted from the body of a patient, transformed and returned to a living body, etc. may be exemplified and any of the methods may be used.

With regard to a method for direct administration to a living body, there is no particular limitation and, for example, it is possible to topically administer into vein, artery, portal vein, cranium, pleura, peritoneum, etc. by means of injection, etc.

Since the dose varies depending upon the type, etc. of protein having RXP repetition at the C-terminal, useful protein, peptide or a plasmid into which gene is inserted, it cannot be determined unconditionally. In the case of adults, however, it is usually preferred to administer 1 to 1,000 mg/kg of a complex. The dose may be appropriately determined by a medical doctor taking the patient's state, condition of the disease, etc. into consideration.

With regard to a method where the target cells are extracted from the body of a patient, transformed and then returned into the body again, firstly the target cells are extracted from the body of a patient. There is no particular limitation for such cells, and examples thereof include bone marrow cell, myeloma cell, liver cell, alveolus cell, muscle cell, lymph cell, lymphoadenoma cell, mammotropic cell, dermatofibroblast, epithelial cell, endothelial cell, interstitial cell, parenchymal cell of organ such as liver, kidney, spleen, thymus, pancreas, placenta, uterus and lung, cartilage cell, nerve cell and the like. With regard to a method for extracting the cells from the body of a patient, there is a method where, for example, body fluid such as blood is collected from the patient, although there is no particular limitation and that may be carried out according to a known method.

Further, the present invention may also be used for various mammalian cells such as HS-68 cell, NIH 3T3 cell, C2C12 cell, CEM-SS cell, HEPG cell, Hela cell, monkey COS-7 cell, Vero cell, Chinese hamster cell CHO (hereinafter, abbreviated as CHO cell), dhfr gene-deficient Chinese hamster cell CHO (hereinafter, abbreviated as CHO(dhfr⁻) cell), mouse L cell, mouse AtT-20 cell, mouse myeloma cell, rat GH3 cell, human FL cell, 293 cell, C127 cell, BALB/3T3 cell, Sp-2 cell, etc.

The above-mentioned cells are proliferated on a medium under a condition for accelerating the proliferation. With regard to the medium, a medium which contains carbon source and nitrogen source may be used. With regard to the carbon source, sugar, organic acid, etc. may be exemplified, and examples of the sugar are glucose, glycerol, starch, dextran, molasses, etc. With regard to the nitrogen source, organic nitrogen source, inorganic nitrogen source, etc. may be exemplified, and examples of the organic nitrogen source are casein, peptone, meat extract, yeast extract, Casamino acid, glycine, etc. and examples of the inorganic nitrogen source are ammonium sulfate, ammonium nitrate, etc.

In order for the transformation to take place, the medium is removed from the cells and the cells are washed with PBS, and then the complex prepared by the above-mentioned condition is added to the desired cells and incubated in a moisturized air containing about 5% of CO₂ for about 1 hour or longer at about 35 to 40°C or, preferably, about 37°C so that the above complex is well contacted to the cells.

After that, about 1 ml of the completely grown medium is added to the cells and incubated at about 15 to 42°C, or preferably about 30 to 40°C for about 0.5 to 2 hour(s) or, preferably, for about 0.5 to 1 hour in the case of useful protein or for about 1 to 2 hour(s) in the case of peptide, low-molecular useful protein or a plasmid. In that case, aeration or stirring may be carried out if desired.

If necessary, a nutrient matter may be added to the medium. Examples of such nutrient matter are serum, amino acid, vitamin, nucleic acid, salt, etc. With regard to the medium, a synthetic medium mainly comprising sugar and inorganic nitrogen source, for example, may be used. When a solid medium is used as a medium, agar is further added thereto.

For the purpose of keeping the transformant stably and suppressing the growth of the strain having no transformant, an antibiotic substance may be added to the medium. Examples of such antibiotic substance are penicillin, erythromycin, neomycin, chloramphenicol, bacitracin, D-cycloserine and ampicillin. Further, anti-foaming agent such as soybean oil, lard oil and various surface-active agents, etc. may be added to the medium if necessary. The pH of the medium is usually about 5 to 9 in view of the pH where the transformant is able to grow, and preferably it is about 6.5 to 7.5.

The resulting transformant is returned to the body of the patient. There is no particular limitation for a method for the process, and it is possible to administer to a patient who needs the treatment by, for example, intravenous administration, topical administration to the diseased part, oral administration, etc. If it is necessary for the purpose of the administration, a pharmaceutically acceptable additive such as carrier, excipient, stabilizer, dissolving aid, or etc. is added thereto to prepare a pharmaceutical preparation suitable for the above-mentioned administration. Incidentally, general procedures for conducting such genetic therapies are fully described, for example, in "Special Issue of *Jikken Igaku;* Biomanual UP Series - Fundamental Technique of Gene Therapy" edited by Takashi Shimada, Izumi Saito and Toshiya Ozawa (1996), Yodosha.

### Examples

### [Test Example 1]

Vero cells which are stable strain of renal cells of African green monkey were grown in an Eagle's minimum essential medium (MEM) containing 5% bovine serum.

In order to check the intercellular transportation ability of HSV-2 protein, plasmid pcDNA-US11 was constructed. PCR primers were US11 Bam HI (ATTAGGATCCATGGCATCCGGGGTTTCC) and US11-R. A PCR product was cleaved by Bam HI and Not I and then cloned by pcDNA 3.1(+). Transfection into the vero cells was conducted according to a protocol recommended by Gibco BRL using a lipofectamine reagent.

Transfection of pcDNA-US11 into the cells was investigated by an indirect immunofluorescence technique using antiserum. The antiserum was produced by conducting immunoreactions by MPL+TDM+CWS emulsion adjuvant system (RIBI ImmunoChem Research, Inc., Montana) using two rabbits and using an emulsion containing about 0.6 mg of HSV US11 protein to which 6xHis expressed in *Escherichia coli* was added. Inoculation was carried out by a hypodermic injection into a shaved back. The same adjuvant and pure protein (0.6 mg) were used for continuous stimulation. Stimulation was applied for three times in total, each stimulation being applied with an interval of two weeks after the first injection. After two weeks from the final immunoreaction, blood was collected from the heart. Rabbit polyclonal anti-US11 antiserum strongly reacts with proteins of apparent molecular weights of 20 kDa and 21 kDa existing in dissolved HSV-2 infected cells.

The cells were washed with PBS and fixed with 4% paraformaldehyde (PFA) at 4°C for 45 minutes. After fixed with PFA, they were washed for 45 minutes with PBS containing 0.05% Triton X-100.

Rabbit polyclonal anti-US11 antiserum was diluted to an extent of 1/500 (w/w) and was used as a primary antibody. After incubating at 37°C for 30 minutes, a cover glass was washed in PBS and labeled with the secondary antibody at 37°C for 30 minutes.

FITC-conjugated goat anti-rabbit IgG (manufactured by MBL) was used as a secondary antibody. After washing with PBS, the cover glass was quickly placed on a slide glass using PermaFluor (manufactured by Immunon). After that, analysis was conducted by a ZEISS laser scanning microscope LSM 510.

Fluorescence of US11 was observed not only in nucleoluses but also in cytoplasms. Moreover, strong fluorescence was also detected in nucleoluses of many cells around them.

### [Test Example 2]

In order to further investigate the spread of HSV US11 protein among the cells, pFLAG-US11 which expresses US11-FLAG fusion protein was constructed and its activity was investigated. In that process, an anti-FLAG mouse monoclonal antibody was used as the primary antibody for the detection of US11-FLAG fusion protein.

In order to prepare pFLAG-US11 which expresses US11-FLAG fusion protein, PCR products of US11GFPFW and US11GFPRv- were introduced into Bgl II/Sal I site of pFLAG-CMV-5a to prepare pFLAG-US11, followed by cloning. Plasmid pFLAG-CMV-5a (Sigma) expresses US11-FLAG fusion protein under the control of HCMV immediate early promoter.

Indirect immunofluorescence measurement was carried out by the same manner as in Test Example 1, except that an anti-FLAG mouse monoclonal antibody (manufactured by Sigma) diluted to an extent of 1/100 (w/w) was used as the primary antibody and FITC-conjugated goat anti-mouse IgG (manufactured by Sigma) was used as the secondary antibody. The anti-FLAG mouse monoclonal antibody recognizes a base sequence comprising eight amino acids (Asp-Try-Lys-Asp-Asp-Asp-Asp-Lys) positioned at KpnI site and thereafter in MCS of a pFLAG-CMV-5a vector.

Both nucleoluses and cytoplasms were strongly stained in small numbers of cells, and further in the cells surrounding them, nucleoluses were stained. Therefore, most of cells showed a nucleolus-staining pattern.

### [Test Example 3]

In order to investigate whether an intercellular transportation ability of HSV US11 protein is maintained when bigger polypeptide is fused, US11-EGFP or EGFP-US11 expression plasmid was constructed.

In order to express a fusion protein of US11 with EGFP, pEGFP-C1-US11 and pEGFP-N3-US11 were constructed. For both of them, the forward PCR primer was US11GFPFW (AATCAGATCTATGGCATCCGGGGTTTCC) in which Bgl II site was integrated. The reverse primer to pEGFP-C1-US11 was US11GFPRv + (AATCGTCGACCTAGGCAAGCCCGCGGGT) and the reverse primer to pEGFP-N3-US11 was US11GFPRv-(AATCGTCGACGGCAAGCCCGCGGGTTGC) integrated in a Sal I site.

A PCR product of each pair was cleaved with Bgl II/Sal I as a Bgl II-Sal I fragment and inserted into pEGFP-C1 and pEFGP-N3 to prepare pEGFP-C1-US11 and pEGFP-N3-US11.

A plasmid was transfected to a cell in the same way as Test Example 1, and 40 hours later, the cell was fixed with cold acetone. Indirect immunofluorescence measurement was carried out by the same manner as in Test Example 1 using a rabbit polyclonal anti-US11 antiserum as a primary antibody and an FITC-conjugated goat anti-rabbit IgG (manufactured by MBL) as a secondary antibody.

In any of US11-EGFP or EGFP-US11, fluorescence was observed in both nucleoluses and cytoplasms in the transfected cells and also in cells surrounding them. Moreover, most of the cells showed a nucleolus-stained pattern. Those result show that intercellular transfection activity among the cells is maintained in those fusion proteins.

### [Test Example 4]

Investigation was carried out to examine whether extrinsic HSV US11 protein was transfected into cells.

US11 fusion protein to which pure 6xHis was added was dialyzed against PBS containing 100 mM NaCl and 10% glycerol and diluted with OPTI MEM (manufactured by Gibco BRL). Vero cells which were a stable strain of renal cells of African green monkey grown in an Eagle's minimum essential medium (MEM) containing 5% bovine serum were washed with PBS for two times. After that, the cells were incubated at 37°C for 10 minutes in the presence of various concentrations of pure HSV US11 protein. After well washed with cold PBS once again, the cells were fixed with cold acetone, and indirect immunofluorescence measurement was conducted by the same manner as in Test Example 1 using a rabbit polyclonal anti-US11 antiserum as a primary antibody and an FITC-conjugated goat anti-rabbit IgG antibody as a secondary antibody.

Although the US11-specific fluorescence was mostly observed in nucleoluses, it was also able to be detected not only in nucleoluses but also in cytoplasms when incubation was conducted in HSV US11 protein having a higher concentration. Those results show that extrinsic HSV US11 protein moves into cells.

### [Test Example 5]

In order to investigate whether an energy-dependent route such as endocytosis participates in internalization of HSV US11 protein in cells, the amount of cells ingested by US11 fusion protein to which 6xHis was added was tested under the condition of 4°C by the same manner as in Test Example 4.

The 6xHis-added US11 fusion protein moves into the cells at 4°C and was found to be accumulated mostly in nucleoluses. Fluorescence of cytoplasms was stronger in the cells incubated at 4°C than those incubated at 37°C. Therefore, it was noted that internalization of the HSV US11 protein in cells was in accordance with an energy-independent route.

### [Example 1]

A protein which induces the production of interferon-γ (hereinafter, abbreviated as IFN-γ) in immune competent cells was used as useful protein. An HSV US11 protein of herpes simplex virus was used as a protein having RXP repetition at the C-terminal.

Pure IFN-γ-producing protein was prepared according to the following method. Dead cells prepared by heating *Corynebacterium parvum* (ATCC 11827) at 60°C for 1 hour were administered by injection into peritoneums of 600 female CD-1 mice of 8 weeks age in an amount of 1 mg per a mouse and, after breeding the mice for seven days by a common and general method, pure lipopolysaccharide derived from *Escherichia coli* was injected into vein in an amount of 1 µg per a mouse. After 1 to 2 hour(s), mice were killed by dislocation of cervical vertebra, blood was collected from the heart, and then the liver was excised, crushed by a homogenizer in 8-fold volume of 50 mM phosphate buffer (pH 7.3) and extracted. The extract was centrifuged at about 8,000 rpm for 20 minutes, 50 mM phosphate buffer (pH 7.3) containing saturated ammonium sulfate was added to about 9 liters of the resulting supernatant liquid so as to make ammonium sulfate 45% saturation, and the mixture was allowed to stand at 4°C for 18 hours and centrifuged at about 8,000 rpm for 30 minutes to give about 19 liters of supernatant liquid containing the protein.

The supernatant liquid was loaded on 4.6 liters of a column of phenyl-Sepharose (manufactured by Pharmacia) which was previously equilibrated with 50 mM phosphate buffer (pH 7.3) containing 1M ammonium sulfate and, after the column was washed with the fresh same buffer, 50 mM phosphate buffer (pH 7.3) was passed through the column at SV 0.57 under a concentration gradient of ammonium sulfate descending from 1M to 0.2M. A fraction (4.8 liters) containing the protein which eluted when the ammonium sulfate concentration was about 0.8 M was collected, subjected to membrane concentration, dialyzed against 20 mM phosphate buffer (pH 6.5) at 4°C for 18 hours, then loaded on a column of 250 mL of DEAE-Sepharose (manufactured by Pharmacia) which was previously equilibrated with 20 mM phosphate buffer (pH 6.5). The column was washed with the fresh same buffer, and then 20 mM phosphate buffer (pH 6.5) was passed through the column at SV 1.2 under a concentration gradient of sodium chloride ascending from 0 M to 0.2 M, whereupon the protein eluted when sodium chloride concentration was about 0.13 M.

The eluate (260 mL) containing the protein was collected, concentrated, dialyzed against 25 mM bis-Tris buffer (pH 7.1) at 4°C for 18 hours and loaded on 24 mL of column of Mono-P (manufactured by Pharmacia) which was previously equilibrated with the fresh same buffer, and 10% (v/v) Polybuffer 74 (pH 4.0) was passed through the column under a pH gradient descending from pH 7 to pH 4, whereupon the protein eluted when pH was 4.8. The eluate (23 mL) containing the protein was collected, concentrated, loaded on a column of Superdex 75 (manufactured by Pharmacia) which was previously equilibrated with a mixed solution (pH 7.2) of 7 mM disodium hydrogen phosphate, 3 mM sodium dihydrogen phosphate and 139 mM sodium chloride, and subjected to a gel filtration chromatography by passing the fresh same mixed solution therethrough, whereupon the protein eluted where the molecular weight was about 19,000 Dalton. A fraction containing the protein was collected. The yield was about 0.6 µg per a mouse.

Pure HSV US11 protein was prepared by the following method. Firstly, a base sequence coding for HSV US11 protein was amplified from HSV-2186 genomic DNA by polymerase chain reaction (hereinafter, referred to as PCR) using US11-F (ATTAGGATCCATGGCATCCGGGGTTTCC) as a forward primer and US11-R (TTATGCGGCCGCCTAGGCAAGCCCGCGGGT) as a reverse primer.

The resulting PCR product was cleaved with Sal I and Not I and inserted into a frame of *Escherichia coli* expression vector pET-28a (manufactured by Novagen), whereupon a plasmid pET-28a-US11 was prepared and then it was cloned. The plasmid was transformed into *Escherichia coli* BL21DE3 strain (manufactured by Novagen), and 6xHis-added US11 fusion protein was expressed in large quantities. The 6xHis-added US11 fusion protein expression cells were dissolved and separated by ultrasonic wave in PBS, cell pieces were removed by centrifugation, and the 6xHis-added US11 fusion protein was obtained by eluting with a 300 mM imidazole-containing fraction.

PBS (5mL) was added to a sterilized tube, and then 50 µL of the HSV US11 protein prepared by the above method and 0.5 µg of IFN-γ-producing protein were added thereto, incubated at 25°C for 30 minutes to give a complex of HSV US11 protein with IFN-γ-producing protein.

### Industrial Applicability

According to the present invention, useful protein, peptide, gene, etc. are efficiently transported to cells in a living body. In addition, according to the present invention, when the useful protein is transported to the cells, it is possible to transport it without causing inactivation or denaturation of the useful protein, and further, even a drug which is difficult to put into cells is able to be easily and directly introduced into the cells, whereby a significant effect of the drug can be achieved.

## Claims

1. A method for transporting at least one of (a) useful protein, (b) peptide and (c) gene in a living body, which is **characterized by** that at least one of (a) useful protein, (b) peptide and (c) gene is/are bound to protein having a structure of peptide unit repetition represented by the formula Arg-Xaa-Pro (in the formula, Xaa is a hydrophobic or acidic amino acid residue) at the C-terminal of the protein and the resulting complex is administered to a living body.

2. The method according to Claim 1, wherein the gene is inserted into a plasmid.

3. The method according to Claim 1, wherein transport in a living body is intercellular transport.

4. The method according to Claim 3, wherein intercellular transport is transport to a target cell.

5. The method according to Claim 4, wherein the target cell is a diseased cell.

6. The method according to Claim 1, wherein the protein is a US11 protein of herpes simplex virus.

7. The method according to Claim 1, wherein the useful protein is a cytokine or interferon-α, -β, -γ or -ω.

8. Use of a protein having a structure of peptide unit repetition represented by the formula Arg-Xaa-Pro (in the formula, Xaa is a hydrophobic or acidic amino acid residue) at the C-terminal of the protein for transporting at least one of (a) useful protein, (b) peptide and (c) gene in a living body.

9. A transport agent for transporting at least one of (a) useful protein, (b) peptide and (c) gene in a living body, which comprises a complex in which protein having a structure of peptide unit repetition represented by the formula Arg-Xaa-Pro (in the formula, Xaa is a hydrophobic or acidic amino acid residue) at the C-terminal of the protein is bound to at least one of (a) useful protein, (b) peptide and (c) gene.

10. A complex in which protein having a structure of peptide unit repetition represented by the formula Arg-Xaa-Pro (in the formula, Xaa is a hydrophobic or acidic amino acid residue) at the C-terminal of the protein is bound to at least one of (a) useful protein, (b) peptide and (c) gene.

11. The complex according to Claim 10, wherein the gene is inserted into a plasmid.

12. The complex according to Claim 10, wherein the protein is a US11 protein of herpes simplex virus.

13. A method for the treatment of human beings or animals, which is **characterized by** that a complex in which protein having a structure of peptide unit repetition represented by the formula Arg-Xaa-Pro (in the formula, Xaa is a hydrophobic or acidic amino acid residue) at the C-terminal of the protein is bound to at least one of (a) useful protein, (b) peptide and (c) gene is administered to a patient.
